## Europäisches Patentamt
## European Patent Office
## Office européen des brevets

(19)

(11) Veröffentlichungsnummer: **0 186 022**
**B1**

(12)

# EUROPÄISCHE PATENTSCHRIFT

(45) Veröffentlichungstag der Patentschrift:
31.05.89

(51) Int. Cl.⁴: **C 07 D 251/10**

(21) Anmeldenummer: **85115610.9**

(22) Anmeldetag: **07.12.85**

(54) **Verfahren zur Herstellung von 2,4-Dioxohexahydro-1,3,5-triazin.**

(30) Priorität: **17.12.84 DD 270913**

(43) Veröffentlichungstag der Anmeldung:
**02.07.86 Patentblatt 86/27**

(45) Bekanntmachung des Hinweises auf die Patenterteilung:
**31.05.89 Patentblatt 89/22**

(84) Benannte Vertragsstaaten:
**AT DE FR GB IT NL SE**

(56) Entgegenhaltungen:
**DD-A-130 479**
**DD-A-138 977**

**CHEMICAL ABSTRACTS, Band 55, Nr. 10, 15. Mai 1961, Columbus, Ohio, USA FRANK B. SLEZAK, ALFRED HIRSCH, LEWIS I. KRIMEN, HENRY A. MC ELRAVY, JR. "Chlorination of 2,4-dioxohexahydro-1,3,5-triazines" Spalte 9411, Zusammenfassung-Nr. 9 411e**
**CHEMICAL ABSTRACTS, Band 73, Nr. 3, 20. Juli 1970, Columbus, Ohio, USA OSTROGOVICH, GEORGE; VIDAC, R.; CATALINA, ELENA "Preparation of methylenediurea and 1,5-methylene-biuret (2,4-doxohexahydro-s-triazine)" Seite 364, Spalte 2, Zusammenfassung-Nr. 14 812k**
**CHEMICAL ABSTRACTS, Band 85, Nr. 19, 8. November 1976, Columbus, Ohio, USA PIKE, RICHARD K. "On the structure of oxonic acid" Seite 523, Spalte 1, Zusammenfassung-Nr. 143 065r**
**CHEMICAL ABSTRACTS, Band 56, Nr. 5, 5. März 1962, Columbus, Ohio, USA A. PISKALA, J. GUT "Nucleic acid components and their analogs. XIII. Synthesis of 5-azauracil (allantoxaidin) and its N-methyl derivatives" Spalte 4766, Zusammenfassung-Nr. 4 766b**
**CHEMICAL ABSTRACTS, Band 101, Nr. 1, 2. Juli 1984, Columbus, Ohio, USA SCHUSTER,**

(73) Patentinhaber: **VEB Leuna- Werke "Walter Ulbricht", DDR- 4220 Leuna 3 (DD)**

(72) Erfinder: **Kiessling, Wolf, Dr., Otto- Adam- Strasse 11, DDR- 7022 Leipzig (DD)**
Erfinder: **Böhm, Günther, Dipl.- Ing., Neustadt, 466/3, DDR- 4090 Halle (DD)**
Erfinder: **Schmidt, Harald, Dr., Dürerstrasse 15, DDR- 4200 Merseburg (DD)**
Erfinder: **Voigt, Dietrich, Dr., Neustadt, 516/2, DDR- 4090 Halle (DD)**
Erfinder: **Baumann, Wolfgang, Göhlitzsch Nr. 6, DDR- 4220 Leuna (DD)**
Erfinder: **Böse, Horst, Dr., Rosenstrasse 48, DDR- 4220 Leuna (DD)**
Erfinder: **Lippert, Bernd, Dipl.- Chem., Neustadt, 233/8, DDR- 4090 Halle (DD)**
Erfinder: **Neumann, Rainer, Dr., Nelkenweg 1, DDR- 4220 Leuna (DD)**
Erfinder: **Hertzer, Werner, Neustadt, 812/2, DDR- 4090 Halle (DD)**
Erfinder: **Zittlau, Helmut, Dipl.- Ing., Willi- Bredel- Strasse 42, DDR- 4020 Halle (DD)**
Erfinder: **Hammer, Lothar, Neustadt, 034/8, DDR- 4090 Halle (DD)**

(74) Vertreter: **Puchberger, Georg, Dipl.- Ing., Patentanwälte Dipl.- Ing. Georg Puchberger Dipl.- Ing. Rolf Puchberger Dipl.- Ing. Peter Puchberger Singerstrasse 13 Postfach 55, A-1010 Wien (AT)**

(56) Entgegenhaltungen: (Fortsetzung)
**GOTTFRIED; ARENHOEVEL, CHRISTOPH "Mode of action of the antiphytoviral compound 2,4-dioxohexahydro-1,3,5-triazine (5-azadihydrouracil)" Seite 199, Spalte 2, Zusammenfassung-Nr. 2 202v**

**Die Akte enthält technische Angaben, die nach dem Eingang der Anmeldung eingereicht wurden**

EP 0 186 022 B1

## Beschreibung

Die Erfindung betrifft die Herstellung von 2,4-Dioxohexahydro-1,3,5-triazin aus Harnstoff und Formaldehyd bzw. aus Formaldehyd enthaltenden Verbindungen und Harnstoff. 2,4-Dioxohexahydro-1,3,5-triazin kann z. B. für die Synthese von Verbindungen mit bakteriziden und fungiziden Eigenschaften genutzt werden oder als langsam wirkendes Stickstoffdüngemittel Verwendung finden. Besonders geeignet ist das nach dem erfindungsgemäßen Verfahren erhaltene Produkt für die Herstellung des Bleichmittelaktivators 1,5-Diacetyl-2,4-dioxohexahydro-1,3,5-triazin.

Es ist bekannt, 2,4-Dioxohexahydro-1,3,5-triazin durch die Umsetzung Formaldehyd enthaltender Verbindungen, wie Methylenbisharnstoff oder Dimethylolharnstoff oder feste Harnstoff-Formaldehyd-Kondensationsprodukte oder Mischungen aus Harnstoff und Paraformaldehyd oder Hexamethylentetramin oder die beim Eindampfen von Harnstoff-Formaldehyd-Lösungen verbleibenden Rückstände, in Gegenwart von soviel Harnstoff, daß im Reaktionsgemisch Molverhältnisse von Formaldehyd zu Harnstoff von 1 : 2,2 bis 1 : 4,5 vorliegen, bei Temperaturen von 373 bis 573 Kelvin 100 - 300° C diskontinuierlich oder kontinuierlich herzustellen (DD-PS-130 479).

Gegenüber der ausschließlichen Verwendung von Methylenbisharnstoff als Rohstoff (DE-PS-694 823, US-PS-3 035 055, US-PS-3 470 175) bietet dieses Verfahren durch den Einsatz überschüssigen Harnstoffes als Reaktionskomponente die Möglichkeit, die Palette der Einsatzprodukte erheblich zu erweitern und Produkte mit verringertem Gehalt an Verunreinigungen zu erhalten.

Nachteilig bleibt, daß die als Feststoffreaktion ablaufende Umsetzung technisch schwer zu beherrschen ist und erheblichen Apparateaufwand erfordert, daß praktisch der gesamte überschüssige Harnstoff in unerwünschte Nebenprodukte übergeführt wird und daß das erfindungsgemäß erhaltene Produktgemisch in einem solchen Umfang verfärbt ist, daß dessen Verwendung zur Herstellung des Bleichmittelaktivators 1,5-Diacetyl-2,4-dioxohexahydro-1,3,5-triazin ohne Reinigung ausgeschlossen werden muß.

Die Verfärbung von 2,4-Dioxohexahydro-1,3,5-triazin kann beispielsweise durch Messung der Extinktion 1,6 %-iger Lösungen in 2 %-iger wäßriger Kalilauge (410 nm, 1 cm-Quarzküvette) quantitativ erfaßt werden. Um 1,5-Diacetyl-2,4-dioxohexahydro-1,3,5-triazin in einer Qualität zu erhalten, die dessen Einsatz in der Waschmittelherstellung ermöglicht, darf die Extinktion der DHT-Lösung den Wert von 0,1 nicht überschreiten. Die nach den bisher bekannten Verfahren zur Herstellung von 2,4-Dioxohexahydro-1,3,5-triazin erhaltenen Produkte genügen dieser Forderung nicht.

Ziel der Erfindung ist die Herstellung von 2,4-Dioxohexahydro-1,3,5-triazin, das ohne weitere Behandlung für die Synthese von 1,5-Diacetyl-2,4-dioxohexahydro-1,3,5-triazin eingesetzt werden kann.

Es bestand die Aufgabe, ein Verfahren zu entwickeln, das die Herstellung von nebenproduktarmen 2,4-Dioxohexahydro-1,3,5-triazin geringer Verfärbung auf einfachem Weg gestattet. Diese Aufgabe wird durch ein Verfahren zur Herstellung von 2,4-Dioxohexahydro-1,3,5-triazin durch Umsetzung von Formaldehyd und Harnstoff bzw. Formaldehyd enthaltenden Verbindungen und Harnstoff in flüssiger Phase bzw. festflüssiger Mischphase erfindungsgemäß dadurch gelöst, daß die Reaktion vor dem Übergang in die feste Phase abgebrochen wird. Vorzugsweise wird die Reaktion nach 70 bis 90 %-igem Umsatz des Formaldehyds zu 2,4-Dioxohexahydro-1,3,5-triazin abgebrochen. Zweckmäßig ist es, wenn die Reaktion durch Zugabe von Wasser abgebrochen wird.

Die erfindungsgemäße Herstellung von 2,4-Dioxohexahydro-1,3,5-triazin ist im allgemeinen so durchzuführen, daß eine wäßrige Formaldehydlösung mit überschüssigem Harnstoff in einer handelsüblichen Rührapparatur ohne Spezialausrüstung auf Reaktionstemperatur erwärmt wird.

Während des Aufheizvorganges reagieren die Einsatzprodukte unter vorzugsweiser Bildung von Methylenbisharnstoff. Das bei der Umsetzung freigesetzte Wasser wird ebenso wie das aus der Formaldehydlösung stammende Wasser abgetrieben, so daß sich im Verlauf des Heizprozesses eine Schmelze bildet, aus der bei Reaktionstemperatur mit steigendem Umsatz des Methylenbisharnstoffes 2,4-Dioxohexahydro-1,3,5-triazin ausgeschieden wird. Entscheidendes Kennzeichen der erfindungsgemäßen Verfahrensführung ist der vor dem Übergang der Schmelze in ein Feststoffgemisch erfolgende Abbruch der Reaktion durch Wasserzugabe.

Die Umsetzung von Harnstoff und Formaldehyd zu 2,4-Dioxohexahydro-1,3,5-triazin kann in einem weiten Temperaturbereich durchgeführt werden. Maßgebend für die Auswahl der Temperatur ist deren Einfluß auf die Reaktionszeit. Während bei niedrigen Temperaturen und langen Reaktionszeiten unbefriedigende Raum/Zeit-Ausbeuten erhalten werden, ist die Synthese bei hohen Temperaturen und sehr kurzen Reaktionszeiten technisch schwer zu beherrschen. Als besonders günstige Temperaturen haben sich unter diesen Gesichtspunkten 443 bis 573 Kelvin (170 - 300° C) erwiesen. In Abhängigkeit von den weiteren Versuchsparametern werden Reaktionszeiten erreicht, die die Herstellung von 2,4-Dioxohexahydro-1,3,5-triazin technisch beherrschbar und ökonomisch effektiv gestalten.

Als Rohstoffe für die erfindungsgemäße Herstellung von 2,4-Dioxohexahydro-1,3,5-triazin sind Harnstoff und Formaldehyd handelsüblicher Qualität geeignet. Dabei ist es prinzipiell ohne Belang, ob Formaldehyd als wäßrige Lösung oder in fester Form eingesetzt wird. Für die technische Durchführung der Reaktion hat es sich als vorteilhaft erwiesen, von wäßrigen Lösungen auszugehen, da bei dieser Verfahrensweise bereits kurz nach Beginn des Aufheizvorganges eine leicht rührbare Reaktionslösung gebildet wird. Es ist auch möglich, Harnstoff und Formaldehyd ganz oder teilweise durch Produkte zu ersetzen, die Harnstoff und Formaldehyd enthalten, wie Methylenbisharnstoff, Methylolharnstoffe, Harnstoff-Formaldehyd-Kondensate oder Hexamethylentetramin, und das gewünschte Molverhältnis gegebenenfalls durch Zusatz von Harnstoff oder Formaldehyd einzustellen.

Das Molverhältnis der Einsatzprodukte ist für die Reaktionsdurchführung von entscheidender Bedeutung, da die Reaktionsschmelze, aus der mit steigendem Umsatz 2,4-Dioxohexahydro-1,3,5-triazin ausgeschieden wird, mit steigendem Harnstoffüberschuß länger fließ- und rührfähig bleibt. Da der überschüssige Harnstoff andererseits in das unerwünschte Nebenprodukt Cyanursäure übergeführt werden kann, ist das Molverhältnis unter Berücksichtigung beider Aspekte auszuwählen. Als besonders geeignet hat sich der Bereich von 0,25 bis 0,35 Mol Formaldehyd pro Mol Harnstoff erwiesen.

Für die Herstellung von 2,4-Dioxohexahydro-1,3,5-triazin nach dem erfindungsgemäßen Verfahren können handelsübliche Rührgefäße eingesetzt werden. Dabei ist die Rühreinrichtung zweckmäßigerweise so zu wählen, daß das Reaktionsgemisch einem Zwangsumlauf unterworfen wird. Durch die Vermeidung fester Phasen ist es nicht erforderlich, komplizierte und kostenaufwendige Spezialapparaturen einzusetzen. Der bis zum Abbruch der Reaktion erreichte Umsatz des Formaldehyds zu 2,4-Dioxohexahydro-1,3,5-triazin ist vom Harnstoffüberschuß, der die Fließfähigkeit des Reaktionsgemisches beeinflußt, abhängig. Aufgrund dieses Zusammenhanges nimmt der in der flüssigen Phase bzw. festflüssigen Mischphase prinzipiell mögliche Formaldehydumsatz zu 2,4-Dioxohexahydro-1,3,5-triazin mit dem Harnstoffüberschuß zu. Im bevorzugten Molverhältnisbereich von 0,25 bis 0,35 Mol Formaldehyd pro Mol Harnstoff erfolgt der Reaktionsabbruch zweckmäßigerweise bei einem Umsatz des Formaldehyds zu 2,4-Dioxohexahydro-1,3,5-triazin von 70 % bis 90 %. Die zum Reaktionsabbruch führende Wasserzugabe ist so durchzuführen, daß Hydrolysevorgänge während der Abkühlphase weitgehend ausgeschlossen werden. Als besonders vorteilhaft hat es sich erwiesen, zwischen dem Vorratsgefäß für Wasser und dem Reaktionsgefäß eine für den schnellen Zufluß ausreichende Druckdifferenz aufzubauen. Das Reaktionsgefäß kann während der Wasserzugabe unter Normaldruck gehalten werden. Bei guter Durchmischung und zügiger Wasserzugabe werden dabei nur sehr geringe Wassermengen abgetrieben.

Die nach der Wasserzugabe erhaltene Suspension wird durch Abtrennung des hauptsächlich 2,4-Dioxohexahydro-1,3,5-triazin enthaltenden Feststoffes aufgearbeitet. In Abhängigkeit von der Reinheit des Produktes und den von der vorgesehenen Verwendung abhängigen Qualitätsforderungen können sich weitere Behandlungen mit Wasser anschließen. Das verfahrensgemäß hergestellte 2,4-Dioxohexahydro-1,3,5-triazin enthält als Nebenprodukt fast ausschließlich Cyanursäure. Andere Verbindungen, wie Methylenbisharnstoff, Biuret und Harnstoff sind im allgemeinen nur in sehr geringen Mengen enthalten. Unlösliche Polymethylenharnstoffe treten praktisch nicht auf. Die Extinktionen 1,6 %-iger Lösungen in 2 %-iger wäßriger Kalilauge (410 nm, 1cm Quarzküvette) liegen im Bereich von < 0,01 bis 0,02.

**Beispiel 1:**

In einen elektrisch beheizbaren Glaskolben von 750 cm$^3$ (ml) Inhalt, der mit Strombrechern, einem Schrägblattrührer, einer Temperaturmessung und einem Destillationsaufsatz ausgerüstet ist, werden 300 g Harnstoff und 141,6 g wäßrige Formaldehydlösung innerhalb von 40 Minuten auf 463 Kelvin (190° C), erwärmt. Das Reaktionsgemisch wird 50 Minuten bei dieser Temperatur gehalten und anschließend durch Zugabe von 400 cm$^3$(ml) Wasser auf ca. 333 Kelvin (60° C) gekühlt. Nach weiterer Kühlung auf 298 Kelvin (25° C) erfolgt die Trennung der gebildeten Suspension durch Filtration des Feststoffes, der zweimal mit je 140 ml Wasser gewaschen wird. Nach der Trennung bei 393 Kelvin (120° C) verbleiben 134,4 g Produkt, das 96,7 % 2,4-Dioxohexahydro-1,3,5-triazin und 3,3 % Cyanursäure enthält. Die auf Formaldehyd bezogene Ausbeute beträgt 64,7 %, die in einer 1 cm-Küvette aus Quarz bei 410 nm gemessene Extinktion einer 1,6 %-igen Lösung in 2 %-iger wäßriger Kalilauge < 0,01.

**Beispiel 2:**

In einem Rührgefäß gemäß Beispiel 1 werden 300 g Harnstoff und 101,6 g 37 %-ige wäßrige Formaldehydlösung innerhalb von 40 Minuten auf 473 Kelvin (200° C) erwärmt. Nach weiteren 35 Minuten bei dieser Temperatur wird das Reaktionsgemisch durch Zugabe von 400 ml Wasser auf ca. 333 K (60° C) abgekühlt. Die Aufarbeitung der Suspension wird gemäß Beispiel 1 durchgefürt. Man erhält 157,1 g Trockenprodukt, das 77,7 % 2,4-Dioxohexahydro-1,3,5-triazin, 16,8 % Cyanursäure sowie 5,5 % eines Gemisches aus Harnstoff, Methylenbisharnstoff, Biuret und nicht identifizierten Verbindungen enthält. Die auf Formaldehyd bezogene Ausbeute beträgt 84,7 %, die gemäß Beispiel 1 gemessene Extinktion < 0,01.

### Beispiel 3:

Die Reaktion wird in einem 100 l Rührgefäß durchgeführt, das einen Schrägblattrührer, Einbauten zur Strombrechung und eine Temperaturmeßstelle enthält. Die Heizung erfolgt über einen Mantel mit Hochdruckdampf. Neben verschiedenen Dosiereinrichtungen sind an das Rührgefäß ein Waschturm mit Wasserkreislauf, ein Wasservorratsgefäß und eine Schälzentrifuge angeschlossen.

Der Reaktor wird mit 50 kg Harnstoff und 20,3 kg einer 37 %-igen wäßrigen Formaldehydlösung beschickt und unter Rühren innerhalb von 70 Minuten auf 458 K (185°C) geheizt. Während des Aufheizvorganges und der Reaktion sammeln sich die aus dem Reaktionsgemisch freigesetzten Verbindungen Wasser, Ammoniak und Kohlendioxid im Kreislaufprodukt des Waschturms. Nach 70 Minuten Reaktionszeit unter Einhaltung einer mittleren Temperatur von 458 K (185°C) wird die Reaktion unter Zugabe von 70 l Wasser aus dem unter einem Druck von 0,1 bis 0,2 MPa. stehenden Vorratsgefäß abgebrochen. Die Suspension kühlt sich auf ca. 333 K (60°C) ab und wird nach weiterer Kühlung auf 298 K(25°C) in 8 Chargen auf einer Schälzentrifuge getrennt. Jede Charge wird zweimal mit je 3 l Wasser gewaschen. Nach der Trocknung bei 353 K (80°C) verblieben 19,1 kg eines Produktes, das 95,9 % 2,4-Dioxohexhydro-1,3,5-triazin und 4,1 % Cyanursäure enthält. Die auf Formaldehyd bezogene Ausbeute beträgt 63,6 %, die gemäß Beispiel 1 gemessene Extinktion < 0,01.

### Beispiel 4:

In einem Reaktor gemäß Beispiel 3 werden 50 kg Harnstoff und 20,3 kg wäßrige Formaldehydlösung innerhalb 70 Minuten auf 458 K (185°C) erwärmt und 100 Minuten bei dieser Temperatur gehalten. Anschließend erfolgt der Reaktionsabbruch durch Zugabe von 70 l Wasser. Die Suspension wird nach Abkühlung auf 298 K (25°C) zentrifugiert, wobei jede der insgesamt 8 Chargen mit 3 l Wasser gewaschen wird.

Nach dem Trocknen bei 353 K (80°C) verbleiben 23,7 kg eines Produktes, das 92,7 % 2,4-Dioxohexahydro-1,3-5-triazin, 6,8 % Cyanursäure und 0,5 % eines Gemisches aus Harnstoff, Methylenbisharnstoff, Biuret und nicht identifizierten Substanzen enthält. Die auf Formaldehyd bezogene Ausbeute beträgt 76,3 %, die gemäß Beispiel 1 gemessene Extinktion 0,01.

### Patentansprüche

1. Verfahren zur Herstellung von 2,4-Dioohexahydro-1,3,5-triazin durch Umsetzung von Formaldehyd und Harnstoff bzw. Formaldehyd enthaltenden Verbindungen und Harnstoff, in flüssiger Phase bzw. festflüssiger Mischphase, dadurch gekennzeichnet, daß die Reaktion vor dem Übergang in die feste Phase abgebrochen wird.

2. Verfahren nach Anspruch 1, dadurch gekennzeichnet, daß die Reaktion nach 70- bis 90 %-igem Umsatz des Formaldehyds zu 2,4-Dioxohexahydro-1,3,5-triazin abgebrochen wird.

3. Verfahren nach den Ansprüchen 1 und 2, dadurch gekennzeichnet, daß die Reaktion durch Zugabe von Wasser abgebrochen wird.

### Claims

1. Process for the preparation of 2,4-dioxohexahydro-1,3,5-triazine by reaction of formaldehyde and urea or formaldehyde containing compounds and urea in a liquid phase or a solid-liquid mixed phase, characterized in that the reaction is discontinued before the transition to the solid phase.

2. Process according to claim 1, characterized in that the reaction is discontinued after a 70 to 90 % conversion of the formaldehyde to 2,4-dioxohexahydro-1,3,5-triazine.

3. Process according to claims 1 and 2, characterized in that the reaction is discontinued by the addition of water.

### Revendications

1. Procédé pour la préparation de la dioxo-2,4 hexahydro triazine-1,3,5 par la réaction de formaldéhyde et d'urée ou de composés contenant du formaldéhyde et d'urée en phase liquide ou en phase solide-liquide mixte, caractérisé en ce que la réaction est arrêtée avant le passage dans la phase solide.

2. Procédé suivant la revendication 1, caractérisé en ce que la réaction est arrêtée après la transformation de 70 à 90 % du formaldéhyde en dioxo-2,4 hexahydro triazine-1,3,5.

3. Procédé suivant les revendications 1 et 2, caractérisé en ce que la réaction est arrêtée par l'addition d'eau.